Europäisches Patentamt

European Patent Office

Office européen des brevets

(19)

(11) Publication number: **0 245 063**
**A1**

(12) **EUROPEAN PATENT APPLICATION**

(21) Application number: **87303976.2**

(22) Date of filing: **01.05.87**

(51) Int. Cl.⁴: **C 12 N 15/00, C 12 N 5/00**

(30) Priority: **01.05.86 GB 8610694**
**30.04.87 ES 870139**

(71) Applicant: **Antibioticos S.A., Bravo Murillo 38, 28015 Madrid (ES)**

(43) Date of publication of application: **11.11.87 Bulletin 87/46**

(72) Inventor: **Jimenez Monton, Antonio, Jose Maranon, 13, ES-28010 Madrid (ES)**
Inventor: **Ortin Monton, Juan, Clara del Rey, 33, ES-28002 Madrid (ES)**

(84) Designated Contracting States: **AT BE CH DE ES FR GB GR IT LI LU NL SE**

(74) Representative: **Ablewhite, Alan James et al, MARKS & CLERK 57/60 Lincoln's Inn Fields, London WC2A 3LS (GB)**

(54) **Eukaryotic resistance markers.**

(57) The present invention relates to prokaryotic puromycin resistance genes used to transform puromycin-sensitive eukaryotic cells to puromycin resistance, to selection techniques involving such transformation, and to eukaryotic cells transformed by such vectors.

EP 0 245 063 A1

M&C FOLIO: 52260                    WANGDOC: 0108C

## Eukaryotic Resistance Markers

The present invention relates to prokaryotic resistance markers for use in transforming eukaryotic cells, to selection techniques involving such markers, and to eukaryotic cells transformed by such markers.

Naked DNA-mediated gene transfer has served as a powerful technique for the isolation of particular genes, for the characterisation of the genetic elements that control gene expression and, in general, for altering the genotype of cells and, ultimately, organisms.

Since transformation of eukaryotic cells was first described in 1973 using the calcium phosphate precipitation technique, methods have improved enormously, with consequent improvement in transformation effiency. However, even using the most advanced techniques, only a small fraction of the recipient cells can be stably transformed.

Accordingly, methods have been developed to enable selection of transformants. Conveniently, this is achieved by labelling transforming DNA with a gene

2

conferring selectable characteristics on the host cell. Such marker genes have traditionally been those encoding enzymes lacking in the recipient, such as HPRT, thymidine kinase, dihydrofolate reductase and APRT. More recently, other genes, such as XGPT, metallothioneine and methotrexate-resistant DHFR, have been employed, as they confer new characteristics on the recipient, rather than rectifying a deficiency, and so are suitable for general usage.

Most commonly-used selection markers for bacteria confer antibiotic resistance. Such markers are generally of little use in eukaryotic selection techniques, as eukaryotic cells do not tend to be sensitive to antibiotics. G418 is one exception to this rule, and a gene encoding resistance to this antibiotic has successfully been used as a selection marker for certain eukaryotic cells (Colbere-Garapin et al., J. Mol. Biol. (1981) 150, 1-14). However, this is currently the only example of such an eukyarotic antibiotic resistance marker, which limits its usefulness, as preferred selection techniques involve at least two resistance markers. A further disadvantage to the use of this marker is the requirement for large quantities of G418 in the selection procedure, due to the relative insensitivity of eukaryotic cells to G418.

It has now been found that certain eukaryotic cell-types are sensitive to the antibiotic puromycin and analogues thereof, such as chryscandin. It has further been discovered that it is possible to tranform relevant cell-types with a Streptomyces resistance gene encoding an aminoglycoside inactivating enzyme, puromycin N-acetyl tranferase (PAC).

To be able to transform eukaryotic cells with DNA from a lower prokaryote and obtain useful expression is especially surprising. It is generally recognised that the genetic expression systems of prokaryotes and eukaryotes vary so widely in their characteristics that, between phylogenic extremes, complete incompatability is the rule.

Streptomyces species employ a different codon system for amino acids to higher prokaryotes and eukaryotes, and have a high GC content in their DNA. They are mycelial gram-positive bacteria, and are significant in that they produce two thirds of known antibiotics. Their mRNAs have a unique 3'-terminal sequence, which probably plays a role in transcription termination. This last feature alone greatly limits the likelihood of obtaining successful expression of a Streptomyces gene in a eukaryotic cell-line.

4

Thus, in a first aspect of the present invention there is provided an eukaryotic expression vector encoding at least the enzyme puromycin N-acetyl transferase, or the enzymatic equivalent thereof.

In an alternative aspect of the present invention, there is provided a method for selecting puromycin sensitive eukaryotic cells transformed with a vector as described above, comprising using puromycin resistance as the selection criterion.

"Puromycin", as used herein, means puromycin and any analogue thereof inactivated by puromycin N-acetyl transferase.

The advantages of the present invention are several-fold. For example, sensitivity of eukaryotic cells to puromycin is substantially greater than to G418, thus requiring reduced amounts of antibiotic for selection. Also, the availability of 2 antibiotic resistance markers enables preferred techniques of selection to be employed. Such techniques may involve a cell-line expressing both markers, transforming the cell-line with the desired DNA, and subsequently selecting for the loss of one of the markers (i.e., the DNA is inserted into a marker gene - "insertional inactivation"). Double transformation is also possible, with its consequent advantages. The ability to assay PAC activity comparatively easily (Vara, J., et al., Biochemistry (1985) 24, 8074-8081) is also to advantage.

As used herein, "PAC" includes fusion proteins and other derivatives incorporating the PAC active site, and "pac" includes genes encoding such proteins and additional sequences as described above.

The pac gene for use in accordance with the present invention is conveniently isolated from S. alboniger. The plasmid pVN3.1 is a particularly useful source of the gene. While Streptomyces is a convenient source of the pac gene, other prokaryotic sources of the gene are of equal use in the present invention.

Only as much of the pac gene as is required to enable synthesis of a protein capable of performing the enzymatic functions of PAC need be incorporated into the expression vector. If expression is restricted to the pac sequence insert, substantially all of the gene may be required to be effective. However, only part, or parts, of the gene may be required if the expressed protein is a fusion protein or the insert is further modified to facilitate/enhance expression. In any event, the minimum pac sequence required is that encoding the PAC active site.

6

Control sequences may be further incorporated into vectors according to the invention as required. Such sequences typically include an rRNA binding site, promoter and termination sequences, and may be those normally associated with the pac gene or, more preferably, will be derived from a eukaryotic system, including viruses such as SV40. Further signal sequences may also be incorporated, such as polyA signals and external secretion signals.

Vectors used to express PAC may be derived from any suitable vector capable of encoding a gene for expression in a eukaryotic host. Such vectors may further encode a known selection marker or markers as appropriate.

Multi-copy plasmids also from a part of the present invention and may enhance expression of PAC. Plasmids wherein the pac gene is present in multiple copies also form a part of the present invention.

The vectors described herein may achieve expression only after inclusion in the genome, or may be able to function as satellite DNA.

Vectors as described above are conveniently plasmids, but may also be any other known suitable expression vehicle.

7

Suitable eukaryotic recipients for use in accordance with the present invention include VERO, protozoan (Vazquez, D., Inhibitors of Protein Biosynthesis. Springer-Verlag, Berlin. (1979)), and insect cells (Falon, A.M. & Stellar, V., Somat. Cell. Genet. (1982) 8, 521-532) and Candida albicans. All of the above are sensitive to puromycin or an analogue thereof which is inactivated by PAC. However, it will be appreciated that the present invention is suitable for use with any eukaryotic cell-type sensitive to puromycin or one of its analogues.

The present invention further includes a eukaryotic cell transformed to expression of PAC by a vector according to the invention, whether transiently, constitutively or controllably.

Variability in transformation efficiency is observed, and the basis for this difference is not entirely clear. It may be due to variable efficiencies in expression of the pac gene, or to differences in the stability of the expressed enzyme, or may be a consequence of the availability of acetyl-CoA (PAC coenzyme) in the cytoplasm of the different cells. Nevertheless, this is the first example of an antibiotic acetyl transferase whose expression can be used as a dominant selectable marker in mammalian cells.

8

The following Examples are for the purpose of illustration only and are not to be construed as limiting the invention in any way.


Example 1: Construction of a PAC-Encoding Plasmid (pSV2pac).


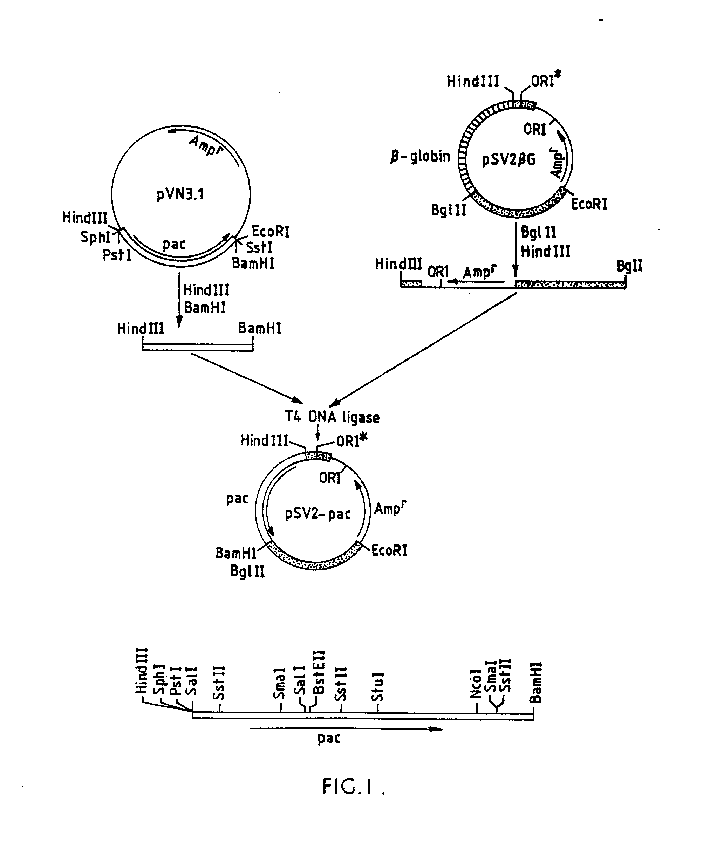

Plasmid pVN3.1 (Fig 1, Wigler, M. et al Proc. Natl. Acad. Sci. USA (1982) 76 1373-1376) carries the pac gene from Streptomyces alboniger. The pac gene was isolated from pVN3.1 as a 1.1 kb Hind111-BamH1 fragment as described by Wigler, M. et al (Proc. Natl. Acad. Sci. USA (1982) 76 1373-1376) and was cloned downstream from the SV40 early promoter, substituting the β-globin gene in the pSV2-β-globin plasmid. The structure of the resulting pSV2βpac plasmid and a detailed restriction map of the pac gene are shown in Figure 1.


· In more detail, pSV2-β-globin and pVN3.1 were digested with restriction endonucleases BglII/HindIII and BamHI/HindIII, respectively. The resulting fragments were separated with low-melting-point agarose. The 4.2 kb fragment from pSV2-βglobin and the 1.1 kb fragment from pVN3.1 were isolated by the CTAB-assisted method (Langridge, J. Anal. Biochem. (1980) 103, 264-271) and ligated T4 DNA ligase. The ligated products were used to transform Escherichia coli

9

AM6, (puromycin-sensitive). Puromycin-resistant (25 µg/ml) colonies were selected on agar plates and plasmid DNA was isolated by the method of Portela, A., et al. (Nuc. Acid Res. (1985) 13, 7913-7927). One of the clones isolated was designated pSV2pac

The lower part of Figure 1 shows a detailed restriction map of the 1.1 kb S. alboniger fragment. The arrows indicate the direction of transcription and the size of the pac or β-lactamase genes. ORI indicates the origin of replication of pBR322 plasmid. ORI* refers to both the origin of replication and early promoter of the SV40 virus. Key to Fig. 1.: (solid line) pBR322, (unshaded box) S. alboniger, (hatched box) β-globin and (stippled box) SV40 DNAs.

A strain of Escherichia coli (AM/139), designated JM83 (pSV2pac) and containing pSV2pac has been deposited under the conditions of the Budapest Treaty at the Torry Research Station, on 17 April, 1986, with accession no. NCIB 12248.

## Example 2: Transient Expression of PAC in Mammalian Cells

The COS-1 cell line was used to test expression of PAC activity in mammalian cells. COS-1 cells constitutively express SV40 T-antigen and allow the transient amplification of SV40-derived replicons, thereby assisting the detection of the expression of cloned genes (Gluzman, Y., Cell (1981) 23, 175-182). Replicate cultures of COS-1 were transfected with pSV2neo or pSV2-pac by the DEAE-dextran procedure described by Lai C.J. & Nathans, (D., Virology, (1974) 60, 466-475). Seventy two hours post-transfection, cells were scraped off the plates, and disrupted by sonication in 100 µl of 50 mM Tris-HCl, 2mM EDTA, 10%(V/V) glycerol, pH 8.5. Enzymic activity was determined spectrophotometrically as described by Vara, J., et al. (Gene, (1985), 33, 197-206).

As shown in Table 1, the cultures transfected with pSV2-pac accumulated the enzyme, whereas the control cells, transfected with pSV2-neo DNA (Southern, P.J. et al., Mol. Appl. Genet. (1982) 1, 327-341) showed no detectable PAC activity.

As the transfection procedure used results in expression of the cloned gene in only a few percent of the cells in the culture (Portela, A. Nuc. Acid. Res. (1985) 13, 7959-7977), the level of PAC accumulation (4-33 mU/mg) observed was similar to the value obtained using Streptomyces sp. and Escherichia coli (Vara, J., etal, Gene, (1985) 33, 197-206), despite the eukaryotic nature of the host cell-line.

11

TABLE 1

| Experiment | Plasmid DNA | Puromycin N-acetyl transferase (mU/mg of protein) |
|---|---|---|
| 1 | pSV2-neo | 0.1 |
| | pSV2-pac | 5.1±0.9 |
| 2 | pSV-neo | 0.1 |
| | pSV-pac | 27.2±5.8 |

## Example 3: Permanent Expression of PAC in Mammalian cells

To determine whether the pac gene could be expressed constitutively in mammalian cells, VERO cell cultures were transformed with a mixture of pSV2-neo and pSV2-pac by the calcium phosphate precipitation technique (Graham, F.L. et al., Virology (1973) 52, 456-467, Wigler, M. et al., Proc. Natl. Acad. Sci. USA (1979) 76, 1373-1376), and clones resistant to G418 selected. Out of 13 clones isolated, one (clone GP1) showed a specific PAC activity of 1.58 mU/mg of protein. This clone was able to grow similarly in the presence of either puromycin (10 μg/ml) or G418 (500 μg/ml), thus proving that the pac gene can be constitutively expressed in cultured mammalian cells.

## Example 4: Use of the pac Gene as a Selection Marker.

A. The usefulness of the pac gene as a selection marker was tested by transformation of VERO cells with pSV2pac DNA, using mock transformation as a control and colonies selected for growth in medium containing puromycin (10µg/ml). No resistant colonies were detected in mock-transformed cultures. Results are shown in Table 2.

Enzymic activity was determined by incorporation of $^{14}$C-acetyl-CoA according to the method of Portela, A., (Nuc. Acid Res. (1985) 13, 7913-7927). Activity values for extracts of VERO cells were below 0.1 mU/mg of protein. Every clone tested expressed measurable PAC activity which correlated with in vivo resistance to puromycin, and contained the pac gene integrated into chromosomal DNA (data not shown).

In Table 2, each cell clone was subcultured with a split ratio of 1:10 into media containing different concentrations of puromycin and incubated at 37°C. (+++), (++), (+), (+/-) and (-) indicate cell growth corresponding to approximately 100, 75, 50, 25 and 0% of that observed in parallel VERO cell cultures, respectively.

Table 2

| Cell clone | PAC activity (mU/mg of Protein) | Growth in puromycin-containing medium(μg/ml) | | | | | |
|---|---|---|---|---|---|---|---|
| | | 0 | 5 | 10 | 20 | 50 | 100 |
| a1 | 14.0 | +++ | +++ | +++ | +++ | +++ | +++ |
| a2 | 3.7 | +++ | +++ | ++ | + | +/- | - |
| a3 | 4.1 | +++ | +++ | +++ | ++ | + | - |
| a6 | 2.8 | +++ | +++ | +++ | ++ | ++ | + |
| E4 | 3.6 | +++ | +++ | +++ | ++ | + | +/- |

B. To test the application of the pac gene as a general dominant selectable marker, cell lines of monkey, hamster, mouse and human origin were used in transformation experiments (pSV2neo was the internal control). To cultures containing a total of 5-17 x $10^6$ cells, puromycin was added to a final concentration of 10μg/ml (HeLa cells received 2.5 μg/ml). After 2-3 weeks, spontaneously resistant colonies were counted and the frequency of spontaneous puromycin resistant mutants was calculated. Results are shown in Table 3.

Two or three dishes of each cell line containing $10^6$ cells were transfected by the calcium phosphate precipitation technique (Graham, F.L. et al., Virology (1973) 52, 456-467, Wigler, M. et al., Proc. Natl. Acad.

14

Sci. USA (1979) 76, 1373-1376) using either 3µg of pSV2-pac or pSV2-neo DNAs. Twentyfour to thirtysix hours thereafter, the cells were subcultured (at a 1:3 ratio for cells receiving pSV2-pac or 1:10 ratio for cells receiving pSV2-neo) and 48 hr later puromycin (10 µg/ml) was added. The media were changed every 3-5 days and 2-3 weeks later the colonies were stained with crystal violet and counted. The numbers indicate the average value of resistant colonies for $2 \times 10^6$ cells obtained in two independent experiments. Every cell line tested was successfully transformed. ND shows that results were not determined.

## TABLE 3

| Cell line | Frequency of spontaneous pur$^r$ mutants $(\times 10^{-6})$ | Number of colonies resistant to | |
|-----------|-----------------------------------------------------------|---------------------------------|------|
| | | puromycin | G418 |
| BHK21 | 0.14 | 284/668 | 1000/1200 |
| VERO | 0.14 | 1/5 | 66/400 |
| L | 4.4 | 19/37 | 162/800 |
| HeLa | 0.2 | 8/ND | 40/ND |

15

## Example 5: Construction of Further PAC-Encoding Plasmids

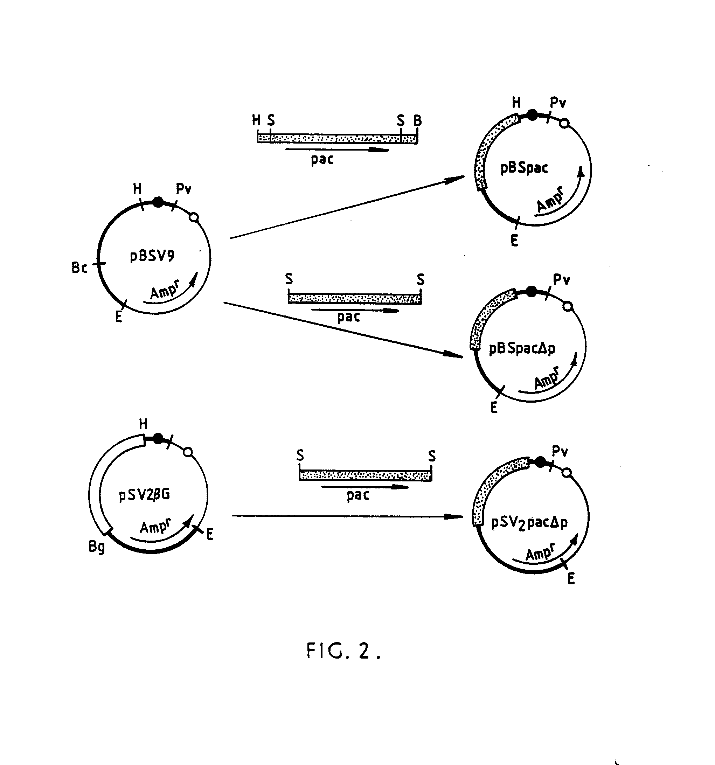

As an alternative to pSV2-β-globin, pBSV9 (Portela et al. (1985) Virus Res. 4, 69-82) was used as parental plasmid to study the effect of eliminating the SV40 small t intron. The 2 inserts used were pac-containing restriction fragments: the 1.1 kbp HindIII-BamHI fragment from pVN3.1 (Vara, J., et al., Nucl. Ac. Res. (1986) 14, 4617-4624) and the 0.9 kbp Sst II subfragment thereof (Fig. 2). The plasmids obtained, pBSpac and pBSpacΔp, lack the SV40 small t intron, while pBSpacΔp also lacks the original prokaryotic pac promoter. The SstII fragment was also used to substitute the β-globin gene in pSV2βglobin, generating pSV2pacΔp, which incorporates the SV40 intron but lacks the pac promoter (Fig 2). The plasmids were obtained by the procedures of Maniatis, T., et al. (Molecular Cloning, A Laboratory Manual, (1982) Cold Spring Harbour Laboratory Press, Cold Spring Harbour, New York).

In Fig. 2., the solid and open circles represent the SV40 and pBR322 origins of replication, respectively. The symbols for restriction nuclease sites area as follows : H. HindIII.; Bc. BcII.; Pv. PvuII.; E. EcoRI.; B. BamHI.; Bg. BglII.; S. SstII, and thin lines represent pBR322 sequences; thick lines SV40

16

sequences; stippled boxes S. alboniger sequences; and unshaded boxed areas represent β-globin gene sequence.

## Example 6: Transient Expression Assays

To study the expression of the pac gene, pSV2pac together with the plasmids described in Example 5, was assayed in a transient expression system (Example 1A). Cultures of COS-1 cells were transfected with the plasmids or mock-transfected (control) and, three days later, low molecular weight DNA was isolated by the method of Hirt, B. et al. (J. Mol. Biol. (1967) 27, 356-369) and analysed by electrophoresis and blot-hybridisation to a pac-specific probe.

Plasmids pSV2pac and pBSpac were found to replicate in COS-1 cells with similar efficiency, while pSV2pacΔp and pBSpacΔp accumulated at five times lower and 50% higher values, respectively.

Plasmid pBSpacΔp replicates better than pSV2pacΔp, possibly due to the presence of pBR322 toxic sequences (Lusky and Botchan, 1981) in the latter. This difference does not affect the pair pSV2pac/pBSpac. The extra Streptomyces sequences flanking the pac gene in pSV2pac apparently enhance replication efficiency compared with pSV2pacΔp.

## Example 7: Accumulation of pac-Specific mRNA

The accumulation of pac-specific mRNA was studied in transfected COS-1 cells by northern analysis of polyA$^+$ RNA isolated three days post-transfection. Size-classes of mRNA of 1850n (pSV2pac), 1650n (pSV2pacΔp), 1170n (pBSpac) and 1000n (pBSpacΔp) were observed (data not shown). This finding is in agreement with the assumption that these mRNAs start at the SV40 early promoter and end at the standard SV40 polyadenylation site.

To determine the relative concentrations of these mRNA species, the corresponding polyA$^+$RNA samples were studied by dot-blot hydridisation. Total cytoplasmic RNA was extracted from cells transfected as above and polyA$^+$ RNA was extracted on oligo-dT-cellulose. Aliquots of 200 ($10^0$) and 20 ($10^{-1}$)ng of polyA$^+$ RNA were fixed to nylon membrane filters (Thomas, P.S., Methods in Enzymology, (1983), 100, 255-266) and hybridised to the pac probe described above. The results (not shown) indicate that those pac mRNAs originating from pBSpac or pBSpacΔp accumulate to levels 5 to 10 times higher than those derived from pSV2pac or pSV2pacΔp (as determined by densitometry).

Since each species derives from the same promoter and the transcription factors are likely to be saturated 18 by the SV40 promoter dose present in the transfected cells, it seems that the presence of the SV40 small t antigen sequences leads to mRNA instability.


Example 8: Expression of PAC


The differences observed in accumulation of pac mRNAs (Example 7) correlate with the levels of PAC activity determined in COS-1 cells transfected with the recombinants by the method described in Example 2. As shown in Table 4 below, recombinants pBSpac and pBSpacΔp induce PAC activities 2-3 times higher than recombinants pSV2pac and pSV2pacΔp. The differences at the protein level are less apparent, and the translation efficiency of the mRNAs derived from pBSpacΔp would appear to be lower than that of the mRNAs obtained from pSV2pac or pSV2pacΔp.


Table 4


PAC activity

| Plasmid construct | Expt 1 | Expt 2 | Expt 3 |
|---|---|---|---|
| | <50 | <50 | <50 |
| pSV2pac | 332 ± 10 | 1036 ± 45 | 1302 ± 380 |
| pBSpac | 1118 ± 758 | 1737 ± 430 | 3073 ± 309 |
| pSV2pacΔp | ND | 1332 ± 210 | 1349 ± 437 |
| pBSpacΔp | ND | ND | 2718 ± 212 |

## Example 9: PAC as a Selection Marker

The higher level of PAC activity in the pBS plasmids was tested for correlation with efficiency as markers for selection for puromycin resistance. Cultures of BHK21, Vero or L cells were transformed with pSV2pac, pBSpac, pSV2pacΔp, pBSpacΔp or mock transformed, by the calcium phosphate precipitation technique (Graham F.L., Virology (1973) 52, 456-467; Wigler M., et al., Proc. Natl. Acad. Sci. USA (1979) 1373-1376) and 24 h later appropriate cell dilutions were plated and selected on puromycin-cotaining media (10μg/ml). Two weeks later, colonies were fixed with formaldehyde, stained with crystal violet and counted. The numbers refer to the resistant colonies obtained from $10^6$ recipient cells.

As shown in Table 5, there is correlation of transformation efficiency and level of PAC activity in transient expression assays.

## Table 5

**Efficiency of Transformation to Puromycin Resistance**

| Plasmid construct | | Number of resistant colonies | | |
|---|---|---|---|---|
| | | BHK21 | Vero | L |
| --- | | 3 | 1 | ND |
| pSV2pac | | 200 | 2 | ND |
| pBSpac | Expt 1 | >3000 | 72 | ND |
| pSV2pacΔp | | >3000 | 45 | ND |
| pBSpacΔp | | ND | ND | ND |
| --- | | 0 | 2 | 8 |
| pSV2pac | | 262 | 10 | 24 |
| pBSpac | Expt 2 | 6255 | 115 | 118 |
| pSV2pacΔp | | 2760 | 85 | 48 |
| pBSpacΔp | | 10275 | 190 | 426 |
| --- | | 0 | 2 | 8 |
| pSV2pac | | 245 | 6 | 27 |
| pBSpac | Expt 3 | 6990 | 105 | 82 |
| pSV2pacΔp | | 2955 | 90 | 28 |
| pBSpacΔp | | 9780 | 225 | 440 |

The differences in transformation efficiencies, compared with PAC activity in the transient assay, may be due to a requirement for multiple integration events in the case of transformation with pSV2pac or

21

pSV2pacΔp, to reach an appropriate PAC activity. Plasmids pBSpac and pBSpacΔp transform $1 \times 10^{-2}$ cells in the BHK21 cell line and $1-4 \times 10^{-4}$ cells in the Vero or L cell lines. These results are comparable to the best efficiencies described for pRSVneo or pRSVgpt (Gorman et al., Science (1983), 221, 551-553).

22

Claims

1. An eukaryotic expression vector encoding at least the enzyme puromycin N-acetyl transferase, or the enzymatic equivalent thereof.

2. A vector according to claim 1 wherein the said enzyme is encoded by genetic material derived from an organism of the genus Streptomyces.

3. A vector according to claim 2 wherein the said organism is S. alboniger.

4. A vector according to any preceding claim having an eukaryotic plasmid as parent vector.

5. A vector according to any preceding claim further comprising sequences affecting expression of the said enzyme.

6. A vector according to claim 5 wherein said sequences comprise at least a promoter and a polyadenylation sequence.

7. A vector as described in any preceding claim comprising at least one further selectable enzyme marker gene.

8. A plasmid selected from the group comprising pSV2pac, pSV2pacΔp, pBSpac and pBSpacΔp.


9. An eukaryotic cell transformed to express puromycin N-acetyl transferase, or the enzymatic equivalent thereof, by a vector as described in any preceding claim.


10. A method for selecting puromycin sensitive eukaryotic cells transformed with a vector according to any of claims 1 to 8, comprising using puromycin resistance as the selection criterion.

FIG.I.

FIG. 2.

**European Patent Office**

**EUROPEAN SEARCH REPORT**

Application number

EP 87 30 3976

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | GENE, vol. 33, no. 2, 1985, pages 197-206, Elsevier Science Publishers, Amsterdam NL; J. VARA et al.: "Cloning and expression of a puromycin N-acetyl transferase gene from Streptomyces alboniger in Streptomyces lividans and Escherichia coli", * Page 198, column 1, lines 18-23; page 204, column 2, line 41 - page 205, line 8 * | 1-10 | C 12 N 15/00 C 12 N 5/00 |
| Y | JOURNAL OF MOLECULAR AND APPLIED GENETICS, vol. 1, no. 4, 1982, pages 327-341, Raven Press, New York, US; P.J. SOUTHERN et al.: "Transformation of mammalian cells to antibiotic resistance with a bacterial gene under control of the SV40 early region promoter" * Whole article * | 1-10 | |
| Y | WO-A-8 203 087 (INSTITUT PASTEUR) * Claims * | 1-10 | |
| | --- -/- | | |

**TECHNICAL FIELDS SEARCHED (Int. Cl.4)**

C 12 N

The present search report has been drawn up for all claims

| Place of search | Date of completion of the search | Examiner |
|---|---|---|
| THE HAGUE | 30-06-1987 | PULAZZINI A.F.R. |

European Patent Office

**EUROPEAN SEARCH REPORT**

Application number

EP 87 30 3976

| | DOCUMENTS CONSIDERED TO BE RELEVANT | | Page 2 | |
|---|---|---|---|---|
| Category | Citation of document with indication, where appropriate, of relevant passages | Relevant to claim | CLASSIFICATION OF THE APPLICATION (Int. Cl.4) | |
| D,P X | NUCLEIC ACIDS RESEARCH, vol. 14, no. 11, June 1986, pages 4617-4624, IRL Press, Oxford, GB; J.A. VARA et al.: "Expression in mammalian cells of a gene from Streptomyces alboniger conferring puromycin resistance" * Whole article * ----- | 1-10 | | |
| | | | TECHNICAL FIELDS SEARCHED (Int. Cl.4) | |

The present search report has been drawn up for all claims

| Place of search | Date of completion of the search | Examiner |
|---|---|---|
| THE HAGUE | 30-06-1987 | PULAZZINI A.F.R. |